# EUROPEAN PATENT APPLICATION

(11) **EP 2 098 251 A1**
(43) Date of publication of application: **09.09.2009**
(21) Application number: 08425143.8
(22) Date of filing: 07.03.2008
(51) Int. Cl.: A61K 51/12

(54) **Method and product for obtaining thin radioactive layers to be used in radiation dosimetry and in medicine**

(71) Applicant: Cipriani, Cesidio, 00185 Rome (IT); Desantis, Maria, 00192 Rome (IT)
(72) Inventor: Cipriani, Cesidio, 00185 Rome (IT); Desantis, Maria, 00192 Rome (IT)

(57) **Abstract**

In the present invention a method and a product useful for the fabrication of radioactive sources in thin layer are proposed. In the resented method differently from the sources currently used in radiation dosimetry and in classical brachytherapy, the obtained sources are mouldable, can easily assume different shapes and thicknesses, can be safety used without contamination. By starting from a solution containing a radioactive isotope, a solid matrix is obtained, in which the radioactive isotope is embedded; the matrix in which the isotope is immobilized is sufficiently flexible and variable to adapt to different surfaces and substrates. The matrix which encloses the radioactive isotope is chosen in order to be chemically compatible with the embedded isotope, and to ensure a homogeneity of the imparted dose. The radioactive sources obtained in this way can be used in the fabrication of calibration sources, and in the field of the therapy of tumours.

## Description

### DESCRIPTION OF THE INVENTION

### Method and product for obtaining thin radioactive layers to be used in radiation dosimetry and in medicine

This invention relates to a method and product for obtaining radioactive sources in the form of thin layer, useful for radiation dosimetry and in radiotherapy. Radioactive sources in thin layer are usually obtained by electrolytic or chemical vapour deposition of a radioactive element on a substrate. In such sources, the distribution of the radioactivity on the substrate material must be uniform, in order to impart a homogeneous dose rate from the surface of the source. In same cases, particularly when alpha or beta emitters are involved, it should be desirable to have sources with low back-scattering, that can be moulded to assume different shapes and thicknesses. The sources can find use in the calibration of technical and scientific instruments, for quality control of radiation detecting instruments, and can particularly be useful in the radiotherapy of superficial tumours. In the past, radioactive sources, in the form of metallic plaques containing the emitters ¹⁰⁶Ru or ⁹⁰Sr, have been used in the brachytherapy of ocular melanoma (Anteby et al., Ann Ophtalmol, 1993, 25(9):339-41). In the present invention a method for the fabrication of radioactive sources in thin layer is proposed. In the proposed method, differently from the sources currently used in radiation dosimetry and in classical brachytherapy with metallic radioactive plaques, the obtained sources are mouldable, to assume different shapes and thicknesses and can be safely used without contamination. By starting from a solution containing a radioactive isotope, a solid matrix is obtained in which the radioactive isotope is embedded; the matrix in which the isotope is immobilized is sufficiently flexible and variable to adapt to different surfaces and substrates. The matrix which encloses the radioactive isotope is chosen in order to be chemically compatible with the embedded isotope, and to ensure homogeneity of the imparted dose. The radioactive sources obtained in this way can be used in the fabrication of calibration sources, but their most efficacious an interesting application is in the field of the therapy of skin tumours. Skin tumour is the most common cancer in humans; the most widespread forms of this class of tumours are melanoma, basal cell carcinoma (BCC), squamous cell carcinoma (SCC). Many different treatments are currently used in the therapy of these tumours, including surgical techniques, topical therapies and classical radiotherapy. The pharmaceutical treatments are used for small, superficial and not recurrent BCC and SCC tumours, but are not indicated for nodular, cystic, infiltrative variants; moreover, recurrence rates from 40% to 20% have been observed, depending from the technique used. The surgery practice is widespread performed, with excision margins of 2-4 mm recommended for well delineated tumours sized up to 2 cm; for those larger than 2 cm, excision with margin of 1 cm, or more is usually suggested, especially for tumours with aggressive course. In spite of that BCC relapses have been observed with tumour presence in the margins, in 33% of the excised cases, while SCC cancers treated with surgery confers a 5-year disease control probability of about 80%. Besides in all cases in which tumours are located in areas on which surgery excision may be very difficult (ear, nose, eyelids, genitals), the aesthetic and functional results are often highly unsatisfactory.

### Proposed technique for the fabrication of radioactive sources

A possible technique for the obtaining of radioactive sources in thin layer can consist in evaporating a volatile solution, containing both a coordination complex of the isotope and a dissolved matrix-forming molecule (i.e. a plastic, or a gum, or a polymer). By this technique, however, a uniformity of dispersion of the isotope during the solvent evaporation is hard to obtain, due to the fact that the evaporation of a solvent is not a regular process. Another possible solution should consist in the use of a polymerizable matrix (epoxy resin, etc.); in this case the semi-solid form of the matrix renders problematic the fabrication of a regular thin layer of uniform thickness. The present patent proposes the use, as a binding matrix, of a paint, preferably chosen in the group of water soluble acrylic paints. Acrylic paint is fast-drying paint, and is a chemically complex mixture, whose main component basically consists in an acrylic polymer emulsion; it also contains variable amounts of thickeners, surfactants, adhesion promoters, preservatives, defoamers, pH modifiers, dispersants, plasticizers. Acrylic paints can be diluted with water, but become water-resistant when dry. Defending on the dilution degree, the finished acrylic painting can resemble a watercolor or an oil painting, or have its own unique characteristics, not attainable with the other products. Acrylic paints have very good adhesive qualities, and are very stable; they resist oxidization and chemical decomposition, and do not yellow over time. Their water-solubility allows for easier cleanup and reduces the need to use chemicals that may create harmful fumes; the material is non toxic and doesn't include in its composition volatile organic solvents. Acrylic paint is also very adhesive and flexible by nature, and can be used on a wide variety of surfaces.

### Example 1 of preparation of a radioactive thin layer

Carrier-free ¹⁸⁸Re as perrhenate was obtained from a ¹⁸⁸W/¹⁸⁸Re generator by elution with saline; the isotope was added to a kit containing 250 mg of thioacetamide, 1 mg of NH₄ReO₄, 1 mg of polyvinilpirrolidone, 100 microliters of conc. HCl, and the solution was heated to 90 °C for 30 minutes. A nanocolloid (tipically 200-800 nm) of Re₂S₇ was obtained; the precipitate was centrifuged and doubly washed by saline, again centrifuged and thoroughly mixed with a synthetic acrylic resin in a test tube, in order to obtain a completely homogeneous distribution of the radioactive ¹⁸⁸Re. The radioactivity of the test tube was measured and the homogeneity in different fractions of the resin was found better than 98%.

### Example 2 of preparation of a radioactive layer

Carrier-added beta emitter isotope ³²P was commercially obtained in the form of sodium phosphate; to 1 ml of the radioactive isotope solution, 1 ml of a 1M solution of BaCl₂ and 0.1 ml of a 1M solution of NH₄OH were added. A precipitate of barium phosphate was formed at room temperature, which was successively centrifuged and doubly washed by saline, again centrifuged and thoroughly mixed with a synthetic acrylic resin in a test tube, in order to obtain a completely homogeneous distribution of the radioactive ³²P. The radioactivity of the test tube was measured, and the homogeneity in different fractions of the resin was found better than 98%.

### Example of application for dosimetry

A uniform distribution of radioactive ¹⁸⁸Re nanocolloid in acrylic resin was obtained, by following the procedure reported in Example 1. A circular source, 1 cm diameter, was prepared by painting the radioactive resin on a solid support. The radioactivity surface distribution of the obtained source was measured by using a phosphor storage screen (Cyclone - Perkin Elmer), in which the integrated density of the screen is proportional to absorbed dose. The source was put in contact with the active screen for a fixed time, interposing between source and screen one or more cellulose acetate sheets, each 100 micron thickness in order to obtain information about the source homogeneity, and the dose distribution curve of beta articles of ¹⁸⁸Re vs depth. The surface homogeneity of the source was found better than 98%. The dose distribution curve obtained by interposing the absorbers between the source and the phosphor storage screen was compared with Montecarlo calculation (EGS4), and with multi-point source calculation. The correlation coefficient between the measured values, and the two above mentioned models was found, respectively, 0.97 and 0.96

### Example of application for radiotherapy

More than 200 patients with histologically confirmed diagnosis of BCC or of SCC were enrolled for brachytherapy treatment, by using the radioactive sources prepared by one of the above mentioned methods. In many of the patients a relapse of the tumour successive to a surgical excision was present; in others a surgical operation would have been impossible, or functionally and/or aesthetically unacceptable. A thorough clinical and dermoscopic examination of the area of the lesion were performed, and histological information was used for estimating the mean depth of tumour, and the limit of the histologically healthy tissue of the lesion. The skin was protected from a possible contamination by a thin layer of a calculated amount of a protective cream, chosen in the group of creams or gels containing one or more hydrophilic components, and the resin was applied above the cream layer. After a few minutes from application the resin solidified, without an appreciable shrinkage or dimensional change. Doses of 40-60 Gy were imparted to depths of 200 - 600 micron from the epidermis, depending from the histological indications. At the edge of the lesion a border of apparently health tissue of some mm was also included in the irradiation area, in order to deposit a homogeneous dose to the tumour cell of the external edge of the lesion. The activity of the beta emitter isotopes used in the treatments ranged from 1mCi in the smaller lesions, up to more than 50 mCi in larger lesions; ⁹⁰Y, ³²P and ¹⁶⁶Ho have been used in some cases, but in 90% of the patients ¹⁸⁸Re was used, due to the superior irradiation properties, availability and favourable chemistry of this isotope. The required application times typically varied from a few minutes to two hours, depending from the applied radioactivity, the size of the lesion, and the calculated dose. After the time necessary to impart the calculated doses, the resin layer has been easily removed by the use of an adhesive tape or plaster directly applied on the radioactive source. All the calculation of dose distribution curve were performed by Montecarlo or point-source numerical calculation methods. Immediately after the irradiation, a mild erythema was visible in the irradiated area, which completely disappeared two from seven days after the treatment. Ten to thirty days after the treatment, the bleeding, often present in the large area lesions, ceased, a scab was formed, and the lesion gradually healed. Most of the patients only needed a single treatment; in some cases two or three treatments were required for a complete healing. In 85% of the treated cases, a complete apparent clinical remission occurred after three to five months, with one single treatment; in the cases in which a second or even a third treatment was performed, a gradual flattening and softening of the lesion was observed, always ending with complete clinical and histological healing in 100% of the treated cases, after a follow up of a mean of four years.

### Advantages of the proposed technique

The classic gamma brachytherapy requires complex programs of treatment planning and calculation of dose distribution, in order to obtain an individually adjusted dose distribution. In spite of the complexity of calculations and irradiation apparatus, the dose distribution is far from ideal, due to the penetrating nature of the photons, and a large number of sessions (30-40) are usually required for a complete treatment. In this respect, the use of beta radiation isotopes embedded in a tailor-made irradiation mould, like the technique proposed in the present patent, can override, in skin tumours, the drawbacks of classical radiotherapy or brachytherapy, which make use for irradiation of gamma or X-rays photons. By using for therapeutic treatments a tailor-made source, like the ones proposed and realized in the present invention, not only the margin of irradiation can be easily controlled by the application of the resin only on the lesion area, but the dose distribution curve obtained by beta particles almost ideally follows the typical distribution of the tumour invasion in the dermal tissue. This fact allows an administration of therapeutic doses only at the required depth, without unnecessary dose deposition in the sub dermal tissue, independently from the lesion shape complexity, and from the number of the lesions. The product can be easily applied by a paint brush on almost every surface (plastic, metal, human tissue, skin, and in general, a wide range of hydrophobic and hydrophilic surfaces) as such, or after dilution with water, always obtaining a regular and smooth surface of uniform thickness. The protection of the skin by a thin layer of an accurately calculated amount of protective cream, chosen in the group of creams or gels containing one or more hydrophilic components, was important to prevent any possible contamination. Moreover, by using the described method, the material of the radioactive source doesn't touch the skin, and the sources are not classified as radiopharmaceutical products. The total dose imparted from the beta emitting layer depends from the amount of radioactive nanocolloid; the mass unitary dose depends from the ratio (radioactivity of colloid)/(weight of resin);the unitary surface dose depends from the ratio (radioactivity of colloid)/(area of applied resin). The amount of resin necessary to a uniform painting is approximately 10 microliters/cm². After the solidification of the resin layer, a second layer, or even a third, can be applied, so increasing the unitary surface dose, and the source thickness. The thickness of the resin must be taken into account for a precise dose evaluation, by calculating the self-absorption effect.

## Claims

1. A method for the fabrication of radioactive sources containing alpha, beta, or gamma emitter isotopes, in thin layer, mouldable in different shapes and thicknesses, that can be safely used without contamination, in radiation dosimetry and in medicine.

2. A product useful for the fabrication of radioactive sources, according to claim 1, consisting in a composite in which the matrix encloses a radioactive isotope uniformly distributed; the matrix must be chemically compatible with the embedded isotope, and must ensure a uniform distribution and homogeneity of the radioactivity.

3. A product useful for the fabrication of radioactive sources, according to claim 1, consisting in a composite in which the matrix enclosing the radioactive isotope is a paint, preferably chosen in the group of water soluble acrylic paints.

4. A product useful for the fabrication of radioactive sources, according to claim 3, in which the embedded isotope is present in aqueous solution, or, more preferably, is present in the form of an insoluble microcolloid or, still more preferably, is present in the form of an insoluble nanocolloid.

5. A product useful for the fabrication of radioactive sources, according to claim 4, in which the embedded isotope is an alpha or a beta emitter.

6. A product useful for the fabrication of radioactive sources, according to claim 5, in which the embedded isotope is preferably the beta emitter emitter isotope ¹⁸⁸Re.

7. A therapeutic kit constituted of the following components: (a) a radioactive isotope, preferably a beta emitter isotope in aqueous solution or dispersion, preferably in the form of an insoluble precipitate, more preferably in the form or an insoluble microcolloid, still more preferably in the form of an insoluble nanocolloid (b) a product that can be mixed in all proportions with the radioactive isotope, chosen in the group of water soluble acrylic paints (c) a cream that can be applied on the skin in thin layers, chosen in the group of creams or gels containing one or more hydrophilic components.

8. A therapeutic kit, according to claim 7, in which the radioactive constituent is ¹⁸⁸Re, preferably in the form or an insoluble microcolloid, still more preferably in the form of an insoluble nanocolloid.

9. A therapeutic kit, according to claim 7, in which the radioactive component and the matrix forming paint can be premixed in every proportion.

10. A therapeutic kit, according to claim 7, comprising all the necessary auxiliary equipment, like a specially designed paint brush, useful for application of the product on the patient lesions, a device useful for the final mixing and homogenizing of the radioactive product and of the matrix forming paint, a program or series of table for dosimetric calculation, a device useful to measure the area of the skin lesions, a shielding device useful to protect critical organs of the patients (crystalline, mucosal tissue) from unwanted irradiation during the therapy, a screen or device useful for the radiation protection of the medical personnel from external irradiation during the phase of application of the product to the patients.
While the description above refers to particular embodiments of the present invention, it should be readily apparent to people of ordinary skill in the art, that a number of modifications may be made without departing from the spirit of the invention. The accompanying claims are intended to cover such modifications as would fall within the true spirit and scope of the invention. The presently disclosed embodiments are, therefore, to be considered in all respects as illustrative and not restrictive, the scope of the invention being indicated by the appended claims rather than the enclosed description. All changes that come within the meaning and range of equivalency of the claims are intended to be embraced therein.
